# EUROPEAN PATENT APPLICATION

(11) **EP 2 422 706 A1**
(43) Date of publication of application: **29.02.2012**
(21) Application number: 11163077.8
(22) Date of filing: 19.04.2011
(51) Int. Cl.: A61B 8/08, G01N 29/44, G01S 7/52

(54) **3D ultrasound system and method for measuring the reflection angles**

(30) Priority: 24.08.2010 KR 20100081995
(71) Applicant: Samsung Medison Co., Ltd., Nam-myun Hongchun-gun Kangwon do 250-875 (KR)
(72) Inventor: Kim, Sung Yoon, 472-735, Gyeonggi-Do (KR)
(74) Representative: Schmid, Wolfgang

(57) **Abstract**

Disclosed are a three dimensional (3D) ultrasound system and a method of operation of the 3D ultrasound system providing a reflection angle of an ultrasonic wave beam. As an example, the ultrasound system may include a probe to sense an ultrasonic wave beam radiated to an object and reflected from the object, and to form a scan region with respect to the object, a reflection angle calculation unit to calculate a reflection angle formed by a reflection of the ultrasonic wave beam at each spot in the scan region, and to store the calculated reflection angle in a table, and a reflection angle display unit to display, based on the table, the reflection angle at a first spot where a point is designated, by interpolating using a point designation order with respect to the scan region.

## Description

### CROSS-REFERENCE TO RELATED APPLICATION

This application claims the benefit of Korean Patent Application No. 10-2010-0081995, filed on August 24, 2010, in the Korean Intellectual Property Office, the disclosure of which is incorporated herein by reference.

### BACKGROUND

### 1. Field of the Invention

The present invention relates to a three dimensional (3D) ultrasound system for providing a direction of an ultrasonic wave beam radiated to an object in a body and reflected, and a method of operating the 3D ultrasound system.

### 2. Description of the Related Art

An ultrasound system may correspond to an apparatus for transferring an ultrasonic signal from a surface of a body to a predetermined portion in the body, that is, an object such as a fetus or an organ, and obtaining an image about a defect of a soft tissue or a bloodstream using information of the ultrasonic signal reflected from a tissue in the body.

Since the ultrasound system is small in size, low in price, capable of displaying an image in real time, and operates without exposing subjects to an X-ray, and the like, the ultrasound system is widely used with other image diagnostic devices such as an X-ray diagnostic device, a computerized tomography (CT) scanner, a magnetic resonance image (MRI) device, a nuclear medicine diagnostic device, and the like.

An image diagnostic device of the ultrasound system may derive an image of the tissue in the body, using the ultrasonic signal. However, since an incidence angle and a reflection angle of the ultrasonic signal with respect to the tissue in the body may not be displayed, the image of the tissue in the body may be examined based on an experience of a tenotomist.

### SUMMARY

An aspect of the present invention provides a three dimensional (3D) ultrasound system and a method of operating the 3D ultrasound system, the 3D ultrasound system displaying a reflection angle formed by a reflection of an ultrasonic wave beam in a region where an object in a body is scanned.

Another aspect of the present invention also provides a 3D ultrasound system and a method of operating the 3D ultrasound system, the 3D ultrasound system displaying a reflection angle formed by a reflection of an ultrasonic wave beam with respect to a spot where a point is designated, in a scan region.

Still another aspect of the present invention also provides a 3D ultrasound system and a method of operating the 3D ultrasound system, the 3D ultrasound system displaying a reflection angle of an ultrasonic wave beam having a relatively large beam magnitude among ultrasonic wave beams sensed with respect to a predetermined spot in a scan region.

A further aspect of the present invention also provides a 3D ultrasound system and a method of operating the 3D ultrasound system, the 3D ultrasound system projecting a scan region onto a contour line region, and displaying a reflection angle based on a virtual contour line configuring the contour line region.

According to an aspect of the present invention, there is provided a 3D ultrasound system, including a probe to sense an ultrasonic wave beam radiated to an object and reflected from the object, and to form a scan region with respect to the object, a reflection angle calculation unit to calculate a reflection angle formed by a reflection of the ultrasonic wave beam at each spot in the scan region, and to store the calculated reflection angle in a table, and a reflection angle display unit to display, based on the table, the reflection angle at a first spot where a point is designated, by interpolating using a point designation order with respect to the scan region.

According to another aspect of the present invention, there is provided a method of operating a 3D ultrasound system, the method including sensing an ultrasonic wave beam radiated to an object and reflected from the object, and forming a scan region with respect to the object, calculating a reflection angle formed by a reflection of the ultrasonic wave beam at each spot in the scan region, and storing the calculated reflection angle in a table, and displaying, based on the table, the reflection angle at a first spot where a point is designated, by interpolating using a point designation order with respect to the scan region.

According to an embodiment, a reflection angle formed by a reflection of an ultrasonic wave beam in a region, on which an object in a body is scanned, may be displayed to be used as a criterion for determining an availability with respect to the scan region.

According to an embodiment, a reflection angle formed by a reflection of an ultrasonic wave beam with respect to a spot, on which a point is designated, in a scan region may be displayed to be used as a criterion for determining an availability with respect to an object image.

According to an embodiment, a reflection angle of an ultrasonic wave beam having a relatively large beam magnitude among ultrasonic wave beams sensed with respect to a predetermined spot in a scan region may be displayed to enhance a data reliability of the reflection angle with respect to the predetermined spot included in the scan region.

According to an embodiment, a scan region may be projected onto a contour line region, and a reflection angle may be displayed based on a virtual contour line configuring the contour line region.

### BRIEF DESCRIPTION OF THE DRAWINGS

These and/or other aspects, features, and advantages of the invention will become apparent and more readily appreciated from the following description of exemplary embodiments, taken in conjunction with the accompanying drawings of which:
FIG. 1 is a diagram illustrating that an ultrasound system radiates an ultrasonic wave beam to a rectangular object, and displays a reflection angle of a reflected ultrasonic wave beam on a plane-shaped scan region and on a fan-shaped contour line region, according to an embodiment of the present invention;
FIG. 2 is a diagram illustrating that an ultrasound system radiates an ultrasonic wave beam to an object in a body, and displays a reflection angle of a reflected ultrasonic wave beam on a plane-shaped scan region and on a fan-shaped contour line region, according to an embodiment of the present invention;
FIG. 3 is a block diagram illustrating an internal configuration of an ultrasound system according to an embodiment of the present invention; and
FIG. 4 is a flowchart illustrating a method of operating an ultrasound system according to an embodiment of the present invention.

### DETAILED DESCRIPTION

Reference will now be made in detail to exemplary embodiments of the present invention, examples of which are illustrated in the accompanying drawings, wherein like reference numerals refer to the like elements throughout. Exemplary embodiments are described below to explain the present invention by referring to the figures.

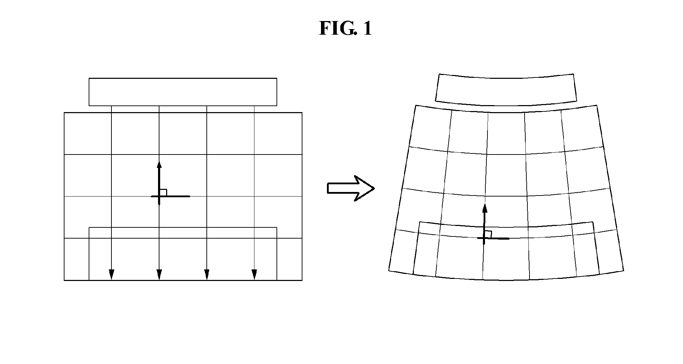

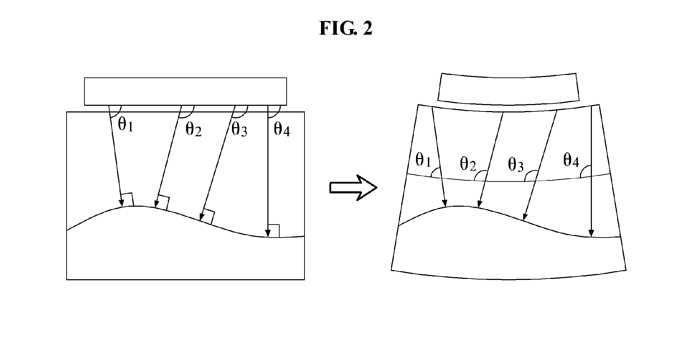

A direction of a beam will be described with reference to FIG. 1 and FIG. 2 illustrating an exemplary example of a reflection angle with respect to an ultrasonic wave beam reflected when an ultrasound system radiates the ultrasonic wave beam on an object or a human body.

FIG. 1 is a diagram illustrating that an ultrasound system radiates an ultrasonic wave beam to a rectangular object, and displays a reflection angle of a reflected ultrasonic wave beam on a plane-shaped scan region and on a fan-shaped contour line region, according to an embodiment of the present invention.

When the ultrasound system radiates the ultrasonic wave beam to the rectangular object, the ultrasonic wave beam may be reflected from the rectangular object to be sensed by a probe. The ultrasonic wave beam sensed by the probe may correspond to a beam reflected from the rectangular object at a right angle. With the ultrasonic wave beam, the ultrasound system may form a scan region with respect to the rectangular object.

The ultrasound system may project and display a plane-shaped scan region onto a fan-shaped contour line region. The ultrasound system may calculate an angle formed by a reflection of the ultrasonic wave beam with respect to the rectangular object in the contour line region, and may store the reflection angle with respect to the scan region in a table. The ultrasound system may store, in the table, a right angle calculated as the reflection angle with respect to the rectangular object.

The ultrasound system may display, based on the table, the reflection angle at a spot designated as a point with respect to a point designation order inputted from an interface terminal.

FIG. 2 is a diagram illustrating that an ultrasound system radiates an ultrasonic wave beam to an object in a body, and displays a reflection angle of a reflected ultrasonic wave beam on a plane-shaped scan region and on a fan-shaped contour line region, according to an embodiment of the present invention.

When the ultrasound system radiates the ultrasonic wave beam to the object in the body through a probe, the ultrasonic wave beam may be reflected from the object to be sensed by the probe. The ultrasonic wave beam sensed by the probe may correspond to a beam reflected from the object at a right angle. Using the ultrasonic wave beam, the ultrasound system may form a scan region with respect to the object in the body.

The ultrasound system may project and display a plane-shaped scan region onto a fan-shaped contour line region. The ultrasound system may calculate an angle formed by a reflection of the ultrasonic wave beam with respect to the object in the body in the contour line region, and may store the reflection angle with respect to the scan region in a table. The ultrasound system may store, in the table, a predetermined angle calculated as the reflection angle with respect to the object in the body.

The ultrasound system may display, based on the table, the reflection angle at a spot designated as a point with respect to a point designation order inputted from an interface terminal.

An internal configuration of the ultrasound system calculating and displaying the reflection angle will be described below.

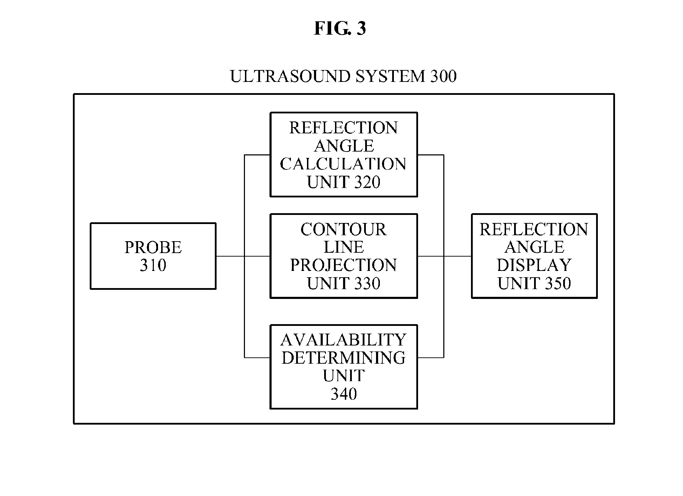

FIG. 3 is a block diagram illustrating an internal configuration of an ultrasound system 300 according to an embodiment of the present invention.

Referring to FIG. 3, the ultrasound system 300 according to an embodiment of the present invention may include a probe 310, a reflection angle calculation unit 320, a reflection angle display unit 350, a contour line projection unit 330, and an availability determining unit 340.

The probe 310 may radiate an ultrasonic wave beam to an object in a body, and may sense an ultrasonic wave beam reflected from the object to form a scan region with respect to the object. The probe 310 may transfer data with respect to the formed scan region to the reflection angle calculation unit 320.

The reflection angle calculation unit 320 may calculate, with reference to the data with respect to the scan region, the reflection angle formed by the reflection of the ultrasonic wave beam at each spot in the scan region, and may store the calculated reflection angle in a table. In this instance, the reflection angle calculation unit 320 may calculate, with respect to a predetermined spot in the scan region, the reflection angle of an ultrasonic wave beam having a relatively large beam magnitude among ultrasonic wave beams sensed by the probe 310.

The contour line projection unit 330 may convert data by projecting the scan region onto a contour line region. The contour line projection unit 330 may convert data by projecting the data in the scan region onto a fan-shaped contour line region.

The reflection angle calculation unit 320 may calculate the reflection angle based on a virtual contour line via a spot where a point is designated, among virtual contour lines configuring the contour line region. The reflection angle calculation unit 320 may calculate the reflection angle between a tangent line of the virtual contour line corresponding to a focal point of the sensed ultrasonic wave beam and an extension line of a focal point of the sensed ultrasonic wave beam.

The reflection angle display unit 350 may display a beam image with respect to the ultrasonic wave beam having the reflection angle as an included angle, based on a virtual contour line via the spot where the point is designated, among virtual contour lines configuring the contour line region.

The reflection angle display unit 350 may display, based on the table formed by the reflection angle calculation unit 320, the reflection angle at the spot where the point is designated, by interpolating using a point designation order with respect to the contour line region where the scan region is projected.

The availability determining unit 340 may determine an availability with respect to data of the scan region based on the reflection angle with respect to the spot where the point is designated, based on data in a gestational age table associated with the object. The availability determining unit 340 may eliminate data of the scan region which differs from a predetermined reflection angle, based on the data in the gestational age table.

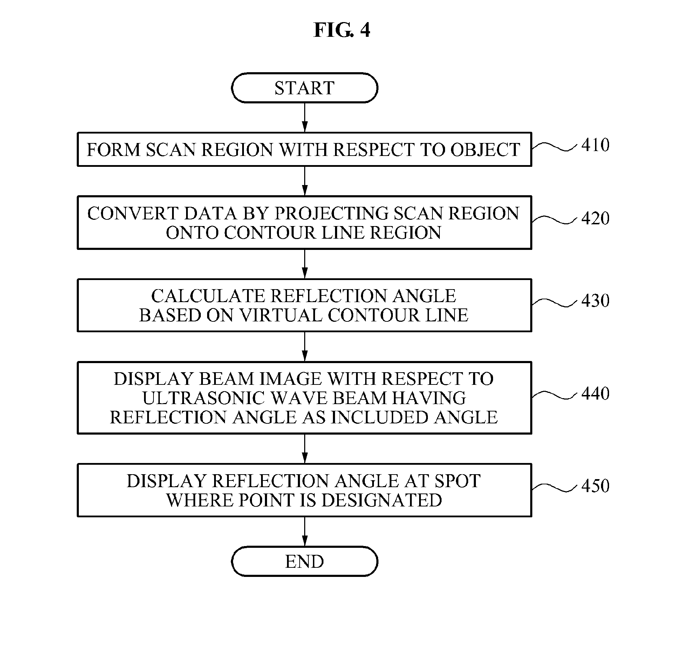

FIG. 4 is a flowchart illustrating a method of operating an ultrasound system according to an embodiment of the present invention.

The method of operating the ultrasound system according to an embodiment of the present invention may be implemented by the ultrasound system 300 illustrated in FIG. 3. Hereinafter, descriptions with reference to FIG. 4 may refer to the above descriptions of FIG. 3.

In operation 410, the ultrasound system may radiate an ultrasonic wave beam to an object in a body, and may sense an ultrasonic wave beam reflected from the object to form a scan region with respect to the object.

The ultrasound system may calculate, with reference to data with respect to the scan region, the reflection angle formed by the reflection of the ultrasonic wave beam at each spot in the scan region, and may store the calculated reflection angle in a table. In this instance, the ultrasound system may calculate, with respect to a predetermined spot in the scan region, the reflection angle of an ultrasonic wave beam having a relatively large beam magnitude among ultrasonic wave beams sensed by a probe.

In operation 420, the ultrasound system may convert data by projecting the scan region onto a contour line region. The ultrasound system may convert data by projecting the data in the scan region onto a fan-shaped contour line region.

In operation 430, the ultrasound system may calculate the reflection angle based on a virtual contour line via a spot where a point is designated, among virtual contour lines configuring the contour line region. The ultrasound system may calculate the reflection angle between a tangent line of the virtual contour line corresponding to a focal point of the sensed ultrasonic wave beam and an extension line of a focal point of the sensed ultrasonic wave beam.

In operation 440, the ultrasound system may display a beam image with respect to the ultrasonic wave beam having the reflection angle as an included angle, based on a virtual contour line via the spot where the point is designated, among virtual contour lines configuring the contour line region.

In operation 450, the ultrasound system may display, based on the table, the reflection angle at the spot where the point is designated, by interpolating using a point designation order with respect to the contour line region where the scan region is projected.

The ultrasound system may determine an availability with respect to data of the scan region based on the reflection angle with respect to the spot where the point is designated, based on data in a gestational age table associated with the object. The ultrasound system may eliminate data of the scan region which differs from a predetermined reflection angle, based on the data in the gestational age table.

The above-described exemplary embodiments of the present invention may be recorded in non-transitory computer-readable media including program instructions to implement various operations embodied by a computer. The media may also include, alone or in combination with the program instructions, data files, data structures, and the like. Examples of non-transitory computer-readable media include magnetic media such as hard disks, floppy disks, and magnetic tape; optical media such as CD ROM disks and DVDs; magneto-optical media such as optical disks; and hardware devices that are specially configured to store and perform program instructions, such as read-only memory (ROM), random access memory (RAM), flash memory, and the like. Examples of program instructions include both machine code, such as produced by a compiler, and files containing higher level code that may be executed by the computer using an interpreter.

Although a few exemplary embodiments of the present invention have been shown and described, the present invention is not limited to the described exemplary embodiments. Instead, it would be appreciated by those skilled in the art that changes may be made to these exemplary embodiments without departing from the principles and spirit of the invention, the scope of which is defined by the claims and their equivalents.

## Claims

1. A three dimensional (3D) ultrasound system, comprising:
a probe to sense an ultrasonic wave beam radiated to an object and reflected from the object, and to form a scan region with respect to the object;
a reflection angle calculation unit to calculate a reflection angle formed by a reflection of the ultrasonic wave beam at each spot in the scan region, and to store the calculated reflection angle in a table; and
a reflection angle display unit to display, based on the table, the reflection angle at a first spot where a point is designated, by interpolating using a point designation order with respect to the scan region.

2. The 3D ultrasound system of claim 1, wherein the reflection angle calculation unit calculates, with respect to a predetermined spot in the scan region, the reflection angle of the ultrasonic wave beam having a relatively large beam magnitude among ultrasonic wave beams sensed by the probe.

3. The 3D ultrasound system of claim 1, further comprising:
a contour line projection unit to convert data by projecting the scan region onto a contour line region,
wherein the reflection angle calculation unit calculates the reflection angle based on a virtual contour line via the first spot, among virtual contour lines configuring the contour line region.

4. The 3D ultrasound system of claim 1, further comprising:
a contour line projection unit to convert data by projecting the scan region onto a contour line region,
wherein the reflection angle display unit displays a beam image with respect to the ultrasonic wave beam having the reflection angle as an included angle, based on a virtual contour line via the first spot, among virtual contour lines configuring the contour line region.

5. The 3D ultrasound system of claim 1, further comprising:
an availability determining unit to determine an availability of the scan region according to the reflection angle with respect to the first spot, based on a gestational age table associated with the object.

6. A method of operating a three dimensional (3D) ultrasound system, the method comprising:
sensing an ultrasonic wave beam radiated to an object and reflected from the object, and forming a scan region with respect to the object;
calculating a reflection angle formed by a reflection of the ultrasonic wave beam at each spot in the scan region, and storing the calculated reflection angle in a table; and
displaying, based on the table, the reflection angle at a first spot where a point is designated, by interpolating using a point designation order with respect to the scan region.

7. The method of claim 6, wherein the calculating comprises calculating, with respect to a predetermined spot in the scan region, the reflection angle of the ultrasonic wave beam having a relatively large beam magnitude among ultrasonic wave beams sensed by the probe.

8. The method of claim 6, further comprising:
converting data by projecting the scan region onto a contour line region,
wherein the calculating comprises calculating the reflection angle based on a virtual contour line via the first spot, among virtual contour lines configuring the contour line region.

9. The method of claim 6, further comprising:
converting data by projecting the scan region onto a contour line region,
wherein the displaying comprises displaying a beam image with respect to the ultrasonic wave beam having the reflection angle as an included angle, based on a virtual contour line via the first spot, among virtual contour lines configuring the contour line region.

10. The method of claim 6, further comprising:
determining an availability of the scan region according to the reflection angle with respect to the first spot, based on a gestational age table associated with the object.
